# EUROPEAN PATENT SPECIFICATION

(11) **EP 3 775 894 B1**
(45) Date of publication and mention of the grant of the patent: **24.04.2024**
(21) Application number: 19799990.7
(22) Date of filing: 13.05.2019
(51) Int. Cl.: G01N 33/53, G01N 33/543, G01N 33/548

(54) **METHOD FOR PREDICTING HUMAN IMMUNE RESPONSE**
VERFAHREN ZUR VORHERSAGE DER MENSCHLICHEN IMMUNANTWORT
MÉTHODE POUR RENFORCER LA RÉPONSE IMMUNITAIRE HUMAINE

(30) Priority: 11.05.2018 US 201862670105 P
(43) Date of publication of application: 17.02.2021
(73) Proprietor: OBI Pharma, Inc., Tapei City 115 (TW)
(72) Inventor: YU, Peiwen, Taipei City 115 (TW); YANG, Ming-Chen, Taipei City 115 (TW); LIN, Yu-Ching, Taipei City 115 (TW); OU, Chien-Chih, Taipei City 115 (TW); YU, Cheng-Der Tony, Taipei City 115 (TW)
(74) Representative: Engelhard, Markus
(86) International application number: PCT/US2019/031960
(87) International publication number: WO 2019/217951

(56) References cited:
- US-A1- 2014 220 601
- US-A1- 2017 304 419
- US-A1- 2017 304 419
- SALETTI GIULIETTA ET AL: "Enzyme-linked immunospot assays for direct ex vivo measurement of vaccine-induced human humoral immune responses in blood", NATURE PROTOCOLS, vol. 8, no. 6, 1 June 2013 (2013-06-01), pages 1073-1087, XP055854810, GB ISSN: 1754-2189, DOI: 10.1038/nprot.2013.058 Retrieved from the Internet: URL:https://www.nature.com/articles/nprot. 2013.058.pdf?proof=tr>
- DEGUCHI TAKASHI ET AL: "Increased Immunogenicity of Tumor-Associated Antigen, Mucin 1, Engineered to Express [alpha]-Gal Epitopes: A Novel Approach to Immunotherapy in Pancreatic Cancer", CANCER RESEARCH, vol. 70, no. 13, 1 July 2010 (2010-07-01), pages 5259-5269, XP055854894, US ISSN: 0008-5472, DOI: 10.1158/0008-5472.CAN-09-4313 Retrieved from the Internet: URL:https://cancerres.aacrjournals.org/con tent/canres/70/13/5259.full.pdf>
- ZHIFANG ZHOU ET AL: "A fully synthetic self-adjuvanting globo H-Based vaccine elicited strong T cell-mediated antitumor immunity", CHEMICAL SCIENCE, vol. 6, no. 12, 1 January 2015 (2015-01-01), pages 7112-7121, XP055458057, United Kingdom ISSN: 2041-6520, DOI: 10.1039/C5SC01402F
- HUANG YEN-LIN ET AL: "Carbohydrate-based vaccines with a glycolipid adjuvant for breast cancer", PROCEEDINGS OF THE NATIONAL ACADEMY OF SCIENCES, NATIONAL ACADEMY OF SCIENCES, US, vol. 110, no. 7, 1 February 2013 (2013-02-01), pages 2517-2522, XP002778272, ISSN: 0027-8424
- LEE HSIN-YU ET AL: "Immunogenicity Study of Globo H Analogues with Modification at the Reducing or Nonreducing End of the Tumor Antigen", JOURNAL OF THE AMERICAN CHEMICAL SOCIETY, AMERICAN CHEMICAL SOCIETY, vol. 136, no. 48, 1 December 2014 (2014-12-01), pages 16844-16853, XP002778274, ISSN: 0002-7863
- TADAO ASAI ET AL: "Evaluation of the modified ELISPOT assay for gamma interferon production in cancer patients receiving antitumor vaccines.", CLINICAL AND DIAGNOSTIC LABORATORY IMMUNOLOGY, 1 January 2000 (2000-01-01), pages 145-154, XP055009957, DOI: 10.1128/CDLI.7.2.145-154.2000
- ASAI et al.: "Evaluation of the Modified ELISPOT Assay for Gamma Interferon Production in Cancer Patients Receiving Antitumor Vaccines", Clin Diagn Lab Immunology, vol. 7, March 2000 (2000-03), pages 145-54, XP055009957, DOI: 10.1128/CDLI.7.2.145-154.2000

## Description

### Related Applications

The present application claims priority of United States provisional application having serial number 62/670,105 filed May 11, 2018.

### Technical Field

The present invention relates to a method for evaluating human immune response to therapeutic cancer vaccine. This method includes a series of culturing procedures and a modified ELISPOT (Enzyme-Linked ImmunoSpot) assay to detect antigen specific antibody producing ability from human individual's PBMC.

### Background of invention

Therapeutic cancer vaccine is an emerging concept that can provide long term protection by stimulating patient's own immune response. Like any other treatments that rely on patient's immune response, such as PD-1/PD-L1, it is crucial to identify responsive population that will benefit from the treatment. In regards to cancer vaccine, whether the patient is able to generate antigen specific antibody would be an important indicator.

Numerous surface carbohydrates are expressed in malignant tumor cells. For example, the carbohydrate antigen Globo H (Fucα1→2 Galβ1→3 GalNAcβ1→3 Galα1→4 Galβ1→4 Glc) was first isolated as a ceramide-linked Glycolipid and identified in 1984 from breast cancer MCF-7 cells. (Bremer E G, et al. (1984) J Biol Chem 259:14773-14777). Previous studies have also shown that Globo H and stage-specific embryonic antigen 3 (Galβ1→ 3GalNAcβ1→ 3Galα1→ 4Galβ1→ 4Glcβ1) (SSEA-3, also called Gb5) were observed on breast cancer cells and breast cancer stem cells (WW Chang et al. (2008) Proc Natl Acad Sci USA, 105(33): 11667-11672). In addition, SSEA-4 (stage-specific embryonic antigen-4) (Neu5Acα2→ 3Galβ1→ 3GalNAcβ1→ 3Galα1→ 4Galβ1→ 4Glcβ1) has been commonly used as a cell surface marker for pluripotent human embryonic stem cells and has been used to isolate mesenchymal stem cells and enrich neural progenitor cells (Kannagi R et al. (1983) EMBO J, 2:2355-2361). These findings support that Globo series antigens (Globo H, SSEA-3 and SSEA-4) are unique targets for cancer therapies and can be used to direct therapeutic agents to targeting cancer cells effectively.

### Summary

One of the objective of the present disclosure is to provide a method to evaluate responsivity of a subject to a carbohydrate antigen or its immunogenic fragment before actually administering the carbohydrate antigen to the subject. Another objective, which is not claimed, is to determine the therapeutic efficacy of a cancer vaccine to a subj ect.

In order to achieve the objective, the present disclosure provides a method of obtaining information that can be used to determine and predict the humoral immune response of patient suspected of having cancer for a carbohydrate antigen, comprising the steps of: (a) obtaining a sample from the patient, (b) cultivating a cell from the sample, (c) exposing the cell *ex vivo* to one or more antigens, (d) identifying the patient is a responder or a non-responder; and (e) continuing a further treatment if the patient is a responder.

The present invention provides a method for evaluating responsivity of a subject to a carbohydrate antigen or its immunogenic fragment, comprising (a) obtaining a peripheral blood mononuclear cell from the subject; (b) exposing the cell ex-vivo with the carbohydrate antigen or its immunogenic fragment in an Enzyme-Linked ImmunoSpot assay performed by using carbohydrate antigen-coated plate; and (c) determining a biological activity according to the assay to evaluate the responsivity, wherein the carbohydrate antigen or its immunogenic fragment is a Globo series antigen, and wherein the carbohydrate antigen-coated plate is a plate without PVDF.

The method of the present disclosure is an *ex-vivo* immunogenicity method on human peripheral blood mononuclear cells (PBMC) to monitor antigen specific antibody production induced by glycan targeting cancer vaccine. The method described here comprises exposure procedures to stimulate PBMC with carbohydrate antigen, following by ELISPOT assay to evaluate responsivity. The spot number can represent the immune response of a subject, which can be used to evaluate the responsivity of the subject to the carbohydrate antigen. SALETTI GIULIETTA ET AL: "Enzyme-linked immunospot assays for direct ex vivo measurement of vaccine-induced human humoral immune responses in blood", NATURE PROTOCOLS,vol. 8, no. 61 June 2013 (2013-06-01), pages 1073-1087, relates to enzyme-linked immunospot assays.

The present disclosure relates to Globo series antigens (Globo H, SSEA-3 and SSEA-4) vaccine, including pharmaceutical compositions comprising adjuvant.

The present disclosure also provides a method for determining the therapeutic efficacy of a cancer vaccine, comprising: (a) providing a sample from a subject; (b) cultivating a cell collected from the sample; (c) contacting the cell and assaying the binding of antibodies bound to the antigens; and (d) determining the therapeutic effect of the antineoplastic agent.

### Brief description of the figures

A more complete understanding of the invention may be obtained by reference to the accompanying drawings, when considered in conjunction with the subsequent detailed description. The embodiments illustrated in the drawings are intended only to exemplify the invention and should not be construed as limiting the invention to the illustrated embodiments.

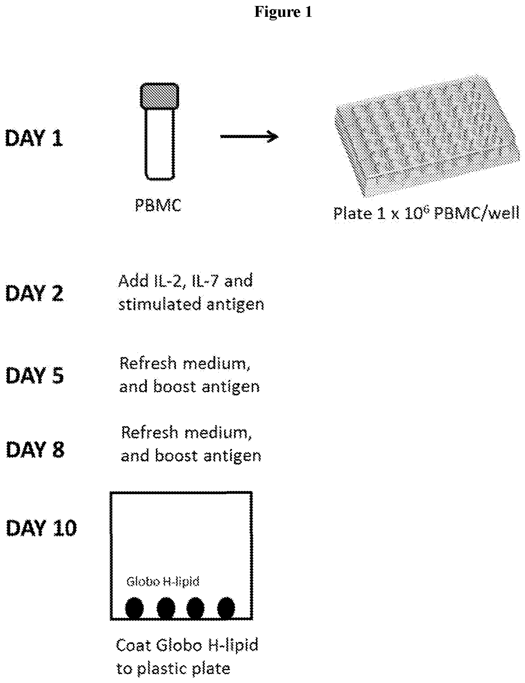

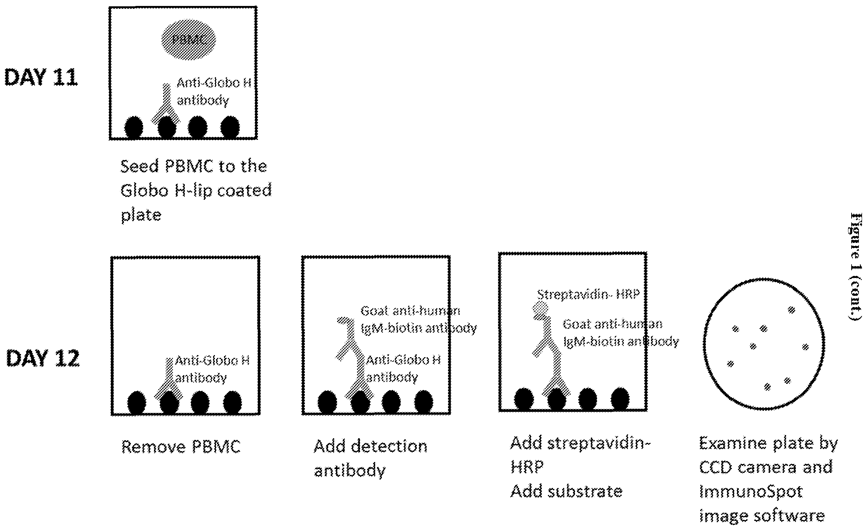

**Figure 1****.** The scheme of the *ex vivo* immunogenicity assay procedures, illustrating cancer vaccine treatment and culturing procedure to un-immunized human peripheral blood mononuclear cells.

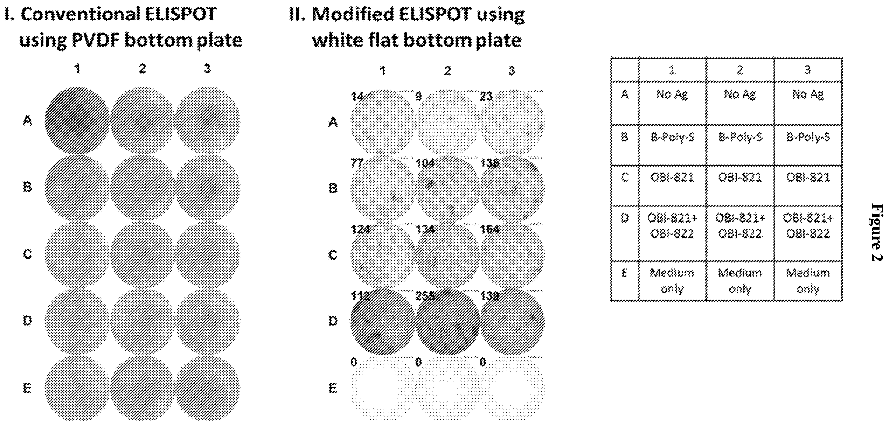

**Figure 2****.** The comparison of conventional and modified anti-Globo H IgM detecting ELISPOT (Group A: No antigen; Group B: B-Poly-S^{™}; Group C: OBI-821 adjuvant; Group D: OBI-821 adjuvant+OBI-822 vaccine; Group E: medium only). Each treatment are seeded in triplicate.

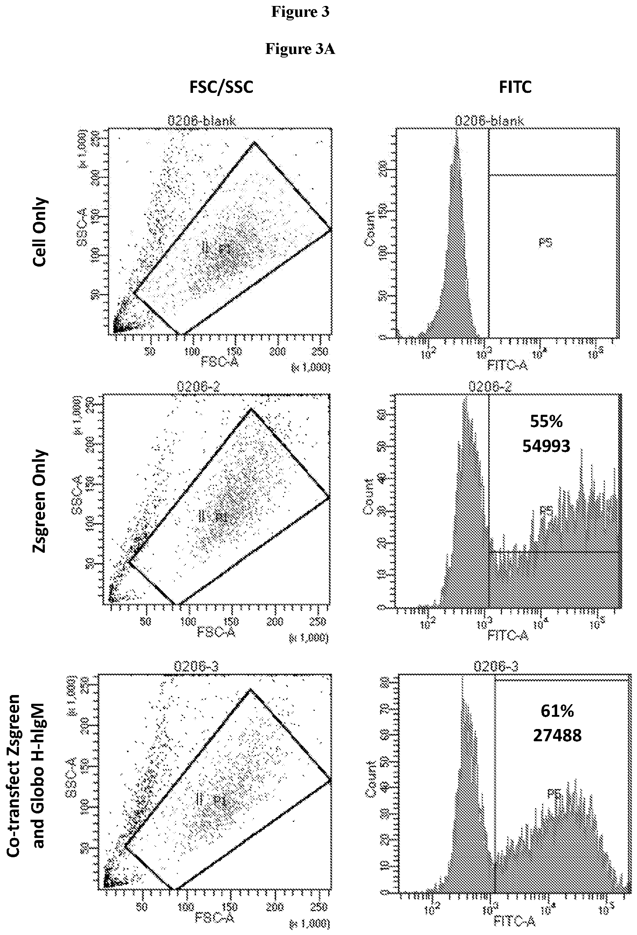

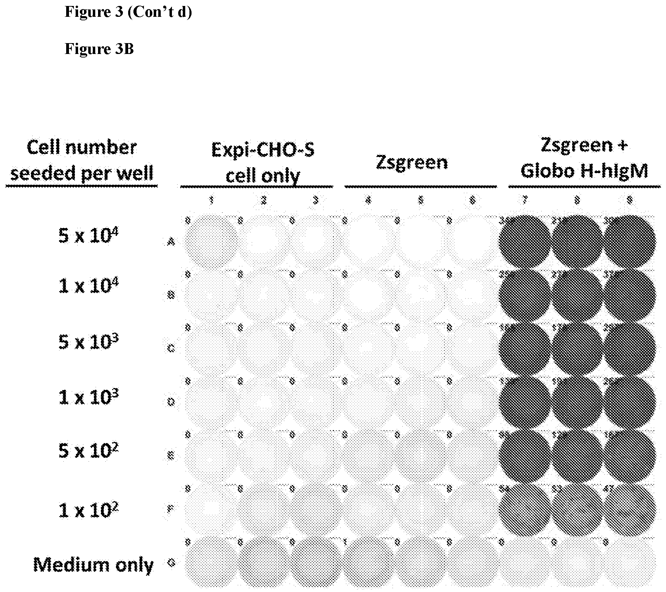

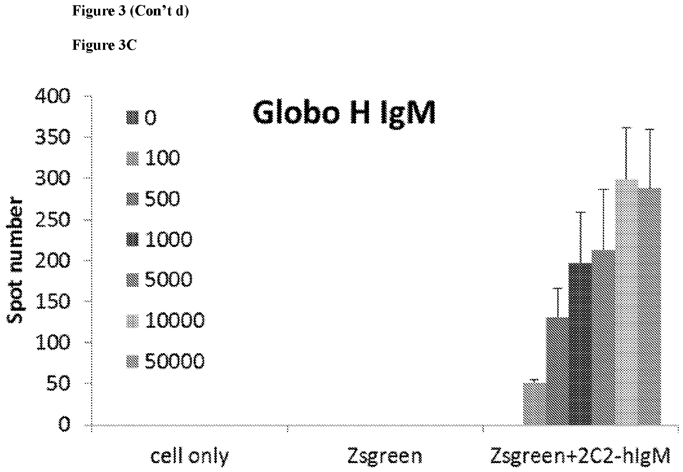

**Figure 3****.** The method accuracy of modified anti-Globo H IgM ELISPOT. **Figure 3A** illustrated the transfection efficiency (PMBC only, Zsgreen only and co-transfect) by flow cytometry analysis on the seeding day (Day 11). **Figure 3B** illustrated anti-Globo H IgM ELISPOT performed with different seeding CHO cell transfections. The seeding CHO cell numbers were ranging from 0 to 5 × 10⁴ cell/per well. Each treatment are seeded in triplicate. **Figure 3C** illustrated the quantitative result of anti-Globo H IgM ELISPOT.

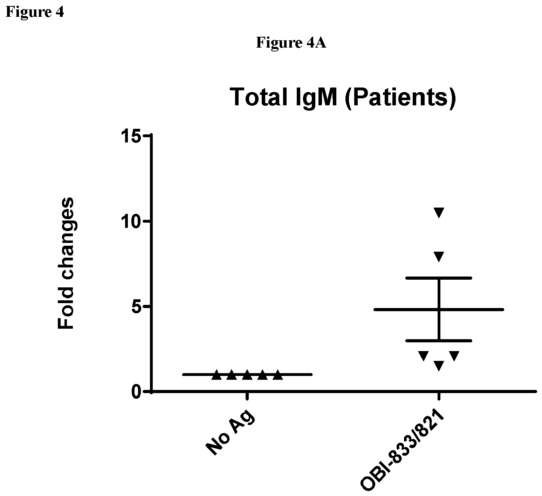

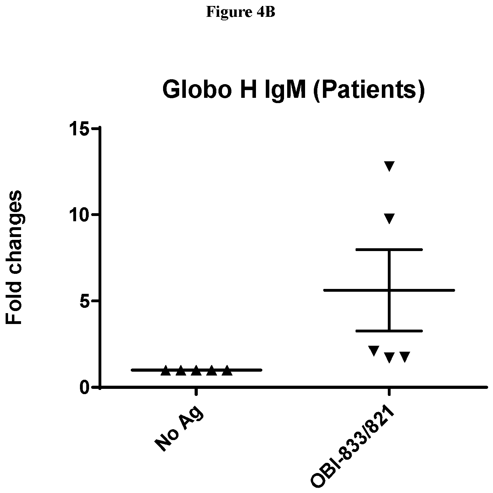

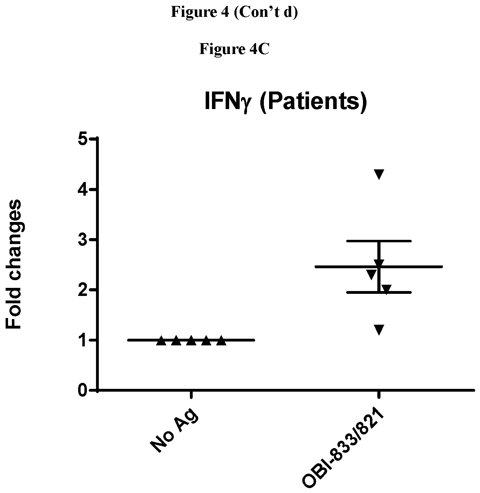

**Figure 4****.** Total IgM, anti-Globo H IgM, and IFN-γ fold change after applying OBI-833/821 immunization procedures to patient PBMC. **Figure 4A****, B, and C** illustrated the fold change of total IgM ELISPOT, anti-Globo H ELISPOT, and IFN-γ induction compared to no-antigen treatment group, respectively.

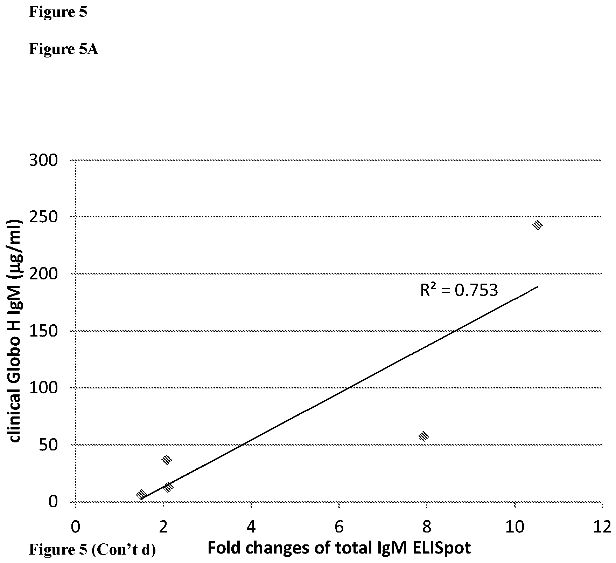

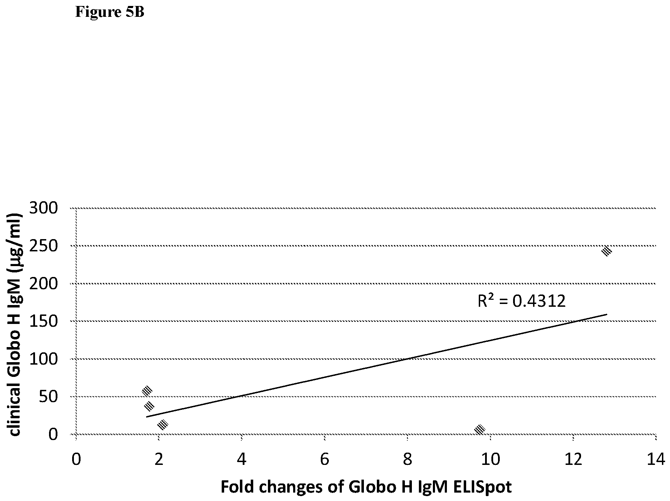

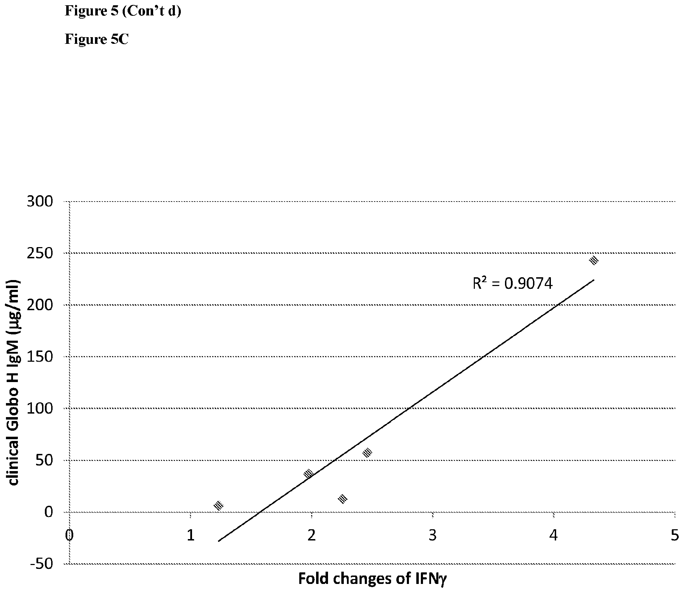

**Figure 5****.** The correlation between *ex vivo* immunogenicity assay results and patient clinical anti-Globo H IgM response. **Figure 5A****, B, and C** illustrated the correlation between total IgM ELISPOT, anti-Globo H IgM ELISPOT, or IFN-*γ* induction and patient maximum anti-Globo H IgM response after OBI-833/821 immunization, respectively.

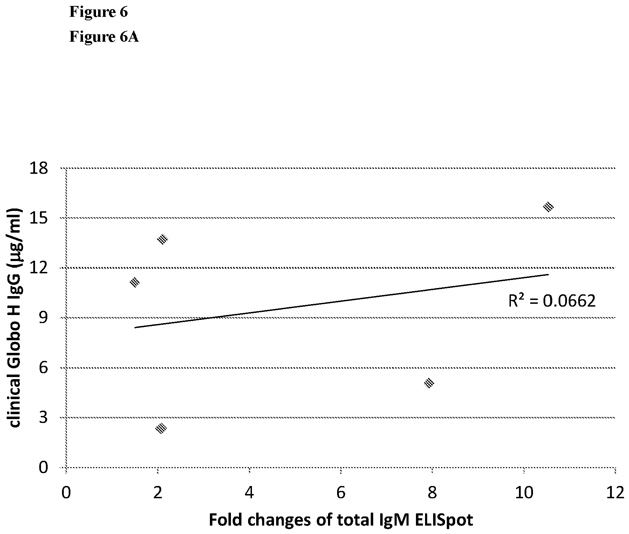

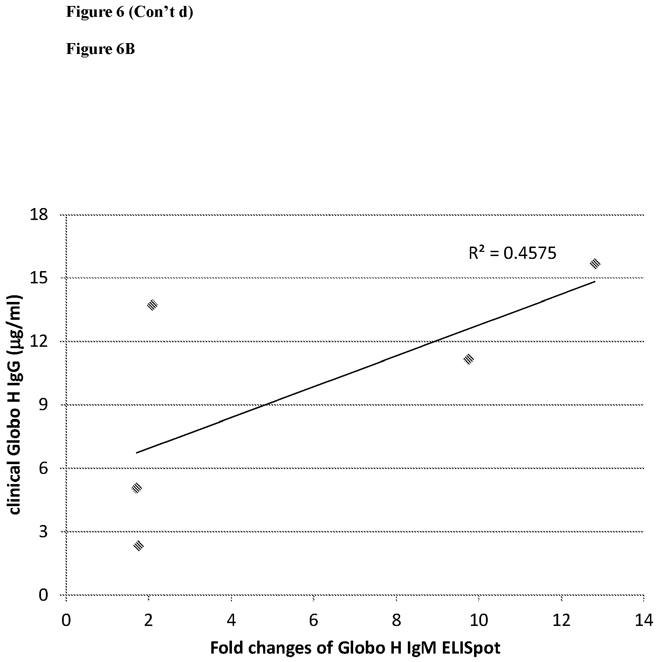

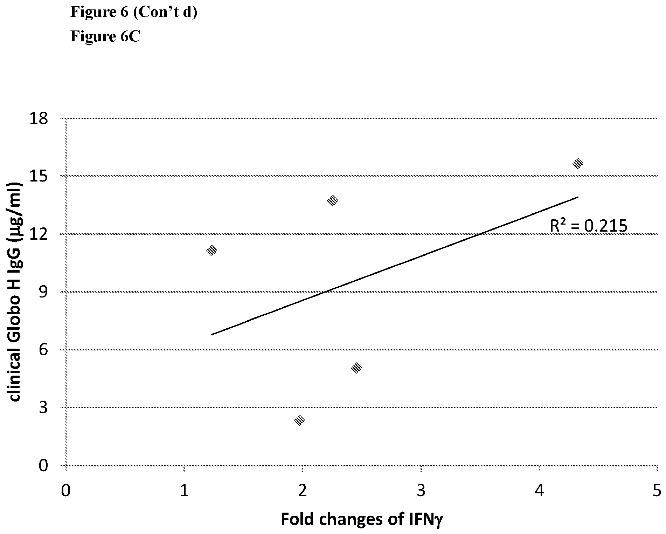

**Figure 6****.** The correlation between *ex vivo* immunogenicity assay results and patient clinical anti-Globo H IgG response. **Figure 6A****, B, and C** illustrated the correlation between total IgM ELISPOT, anti-Globo H IgM ELISPOT, or IFN-γ induction and patient maximum anti-Globo H IgG response after OBI-833/821 immunization, respectively.

### Detailed description of the invention

For the descriptions herein and the appended claims, the singular forms "a", and "an" include plural referents unless the context clearly indicates otherwise. Thus, for example, reference to "a protein" includes more than one protein, and reference to "a compound" refers to more than one compound. The use of "comprise," "comprises," "comprising" "include," "includes," and "including" are interchangeable and not intended to be limiting. It is to be further understood that where descriptions of various embodiments use the term "comprising," those skilled in the art would understand that in some specific instances, an embodiment can be alternatively described using language "consisting essentially of' or "consisting of."

Disclosed is a method of obtaining information that can be used to determine and predict the humoral immune response of patient suspected of having cancer for a carbohydrate antigen, comprising the steps of: (a) obtaining a sample from the patient, (b) cultivating a cell from the sample, (c) exposing the cell *ex vivo* to one or more antigens, (d) identifying the patient is a responder or a non-responder; and (e) continuing a further treatment if the patient is a responder.

The present disclosure discloses a method for evaluating responsivity of a subject to a carbohydrate antigen or its immunogenic fragment. The method comprises a. obtaining a peripheral blood mononuclear cell from the subject; b. exposing the cell *ex-vivo* with the carbohydrate antigen or its immunogenic fragment in an Enzyme-Linked ImmunoSpot assay performed by using carbohydrate antigen-coated plate; and c. determining a biological activity according to the assay to evaluate the responsivity.

The present disclosure also provides a method for determining the therapeutic efficacy of a cancer vaccine, comprising: (a) providing a sample from a subject; (b) cultivating a cell collected from the sample; (c) contacting the cell and assaying the binding of antibodies bound to the antigens; and (d) determining the therapeutic effect of the antineoplastic agent.

The term "patient" used herein is not limited to a person who has the disease (ex. cancer) for sure but also includes those who are suspected of having the disease. In a preferable embodiment, the further treatment could involve the usage of the antigen (carbohydrate antigen).

The term "responder" used herein is referred to a person who is in response to the exposure of the carbohydrate antigen to an extend that exceeds a threshold value. The threshold value (which in some circumstance can be considered as a cut-off value) could vary depending on the type of disease, the average biological activity of a peer group, the medical practitioner's opinion, etc. The tem of "non-responder" is defined on the contrary of "responder" as above.

The term "responsivity" is referred to the degree of the subject in response to the exposure of the carbohydrate antigen. In an embodiment, the responsivity represents a biological activity induced directly or indirectly by the carbohydrate antigen. The term "biological activity" can be defined by a biological change in the product of any effector molecules, including but not limited to IgM, IgM against the carbohydrate antigen, cytokine, or a combination thereof. In an embodiment, the responsivity can have therapeutic meaning. In a specific embodiment, the responsivity can be applied to determine the therapeutic efficacy of the carbohydrate antigen to the subject. In a preferable embodiment, the responsivity is evaluated by comparing the biological activity with a threshold value and the subject is identified as responsive to the carbohydrate antigen if the biological activity exceeds the threshold value. In a specific embodiment, the biological activity is determined by spot number of the Enzyme-Linked ImmunoSpot assay.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-10, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

In an embodiment, the carbohydrate antigen-coated plate is a plate without PVDF. In a specific embodiment that the carbohydrate antigen is a Globo series antigen, the carbohydrate antigen-coated plate is a Globo series antigen-lipid coated plate. In an exemplary embodiment, the carbohydrate antigen-coated plate is commercially available flat white plate.

In a preferable embodiment, the carbohydrate antigen or its immunogenic fragment is a Globo series antigen. Numerous surface carbohydrates are expressed in malignant tumor cells. For example, the carbohydrate antigen Globo H (Fucα1→2 Galβ1→3 GalNAcβ1→3 Galα1→4 Galβ1→4 Glc) was first isolated as a ceramide-linked Glycolipid and identified in 1984 from breast cancer MCF-7 cells. (Bremer E G, et al. (1984) J Biol Chem 259: 14773-14777). Previous studies have also shown that Globo H and stage-specific embryonic antigen 3 (Galβ1→ 3GalNAcβ1→ 3Galα1→ 4Galβ1→ 4Glcβ1) (SSEA-3, also called Gb5) were observed on breast cancer cells and breast cancer stem cells (WW Chang et al. (2008) Proc Natl Acad Sci USA, 105(33): 11667-11672). In addition, SSEA-4 (stage-specific embryonic antigen-4) (Neu5Acα2→ 3Galβ1→ 3GalNAcβ1→ 3Galα1→ 4Galβ1→ 4Glcβ1) has been commonly used as a cell surface marker for pluripotent human embryonic stem cells and has been used to isolate mesenchymal stem cells and enrich neural progenitor cells (Kannagi R et al. (1983) EMBO J, 2:2355-2361). These findings support that Globo series antigens (Globo H, SSEA-3 and SSEA-4) are unique targets for cancer therapies and can be used to direct therapeutic agents to targeting cancer cells effectively.

In a preferable embodiment, DT is diphtheria toxin cross-reacting materials (DT-CRM) or diphtheria toxoids. A DT-CRM refers to a mutant diphtheria toxin, e.g., by mutation or by chemical modification, such that it no longer possesses sufficient ADP-ribosyl. Non limiting examples of DT-CRM include DT-CRM 30, DT-CRM 45, DT-CRM 176, DT-CRM 197 and DT-CRM 228. A diphtheria toxoid is a formaldehyde-inactivated diphtheria toxin. DT is commercially available from or can be prepared by methods known in the art, such as recombinant DNA technology as described in U.S. Patent No. 5,614,382.

In a specific embodiment, the carbohydrate antigen or its immunogenic fragment is conjugated with a carrier protein; wherein, the carrier protein can be, but not limited to KLH (Keyhole limpet hemocyanin), DT-CRM 197 (diphtheria toxin cross-reacting material 197), or a combination thereof.

Practically, the carbohydrate antigen might be formulated with an adjuvant into a cancer vaccine. The adjuvant comprises, but not limited to, saponin, Freund's adjuvant, α-galactosyl-ceramide (α-GalCer) adjuvant, aluminum phosphate, aluminum hydroxide, MF59 (4.3% w/v squalene, 0.5% w/v polysorbate 80 (Tween 80), 0.5%> w/v sorbitan trioleate (Span 85)), CpG-containing nucleic acid, QS21 (saponin adjuvant), a -Galactosyl-ceramides or synthetic analogs thereof (e.g., C34, see US 8,268,969), MPL (Monophosphoryl Lipid A), 3DMPL (3-O-deacylated MPL), extracts from Aquilla, ISCOMS (see, e.g., Sjolander et al. (1998) and J. Leukocyte Biol. 64:713), LT/CT mutants, poly(D,L-lactide-co-glycolide) (PLG) microparticles, Quil A, QS-21 (a saponin from *Quillaja saponaria* tree bark), interleukins, Freund's, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-( -2'-dip-almitoyl-sn-glycero-3-hydroxyphosphoryloxy)-ethylamine (CGP 19835 A, referred to as MTP-PE), and RIBI, which contains three components extracted from bacteria, monophosphoryl lipid A, trehalose dimycolate and cell wall skeleton (MPL+TDM+CWS) in a 2%> squalene/Tween 80 emulsion, or a combination thereof.

The cancer vaccine is applied for preventing or treating Globo series antigen expressing cancer including, but are not limited to, sarcoma, skin cancer, leukemia, lymphoma, brain cancer, glioblastoma, lung cancer, breast cancer, oral cancer, head-and-neck cancer, nasopharyngeal cancer, esophagus cancer, stomach cancer, liver cancer, bile duct cancer, gallbladder cancer, bladder cancer, pancreatic cancer, intestinal cancer, colorectal cancer, kidney cancer, cervix cancer, endometrial cancer, ovarian cancer, testical cancer, buccal cancer, oropharyngeal cancer, laryngeal cancer and prostate cancer.

Specifically, the cancer vaccine can be OBI-822/821, OBI-833/821, OBI-833/834, OBI-866/821 or OBI-866/834. Exemplary OBI-822 (Globo H-KLH glycoconjugate) is as described in WO2015/159118, WO2016/044326 and WO2017/185089 patent applications. Exemplary OBI-833 (Globo H-DT glycoconjugate) is as described in WO2010/005598 and WO2014/107652 patent applications, the content of which is incorporated by reference in its entirety. Exemplary OBI-866 (SSEA4-KLH glycoconjugate) is as described in WO2018/022933 patent application. Exemplary OBI-821 is a saponin adjuvant and OBI-834 is a α-galactosyl-ceramide (α-GalCer) adjuvant.

In certain embodiment, the therapeutic cancer vaccine is OBI-822 (Globo H-KLH glycoconjugate, OBI Pharma, Inc.) combined with adjuvant OBI-821 (saponin adjuvant, OBI Pharma, Inc.) were treated to the human PBMC for their immunogenicity ability test, especially the ability of antigen specific anti-Globo H IgM and IgG induction.

In certain embodiment, the therapeutic cancer vaccine is OBI-833 (Globo H-DT glycoconjugate, OBI Pharma, Inc.) combined with adjuvant OBI-821 (saponin adjuvant, OBI Pharma, Inc.) were used to treat the human PBMC to observe their ability to stimulate the production of immunoglobulins, especially the ability of antigen specific anti-Globo H IgM and IgG induction.

The sample is a body fluid (serum, saliva, lymph node fluid, urine, vaginal swab, or buccal swab) collected from patient. In an embodiment, the term "vaccine", "cancer vaccine" and "antineoplastic agent" are interchangeable in this specification.

### Examples

Various features and embodiments of the disclosure are illustrated in the following representative examples, which are intended to be illustrative, and not limiting. Those skilled in the art will readily appreciate that the specific examples are only illustrative of the invention as described more fully in the claims which follow thereafter.

### Example 1: The scheme of experimental procedure

### Culture of human PBMC

The overall treatment procedure scheme is shown in **Figure 1****.** Frozen vial of human PBMC, either from healthy donor or harvested form patients, was thawed and immediately washed once by complete culture medium (RPMI-1640 with 10% FBS, 1% sodium pyruvate, 1% L-glutamine, 0.1% 2-ME, 1% non-essential amino acid, and 1% penicillin-streptomycin). Cell pallet was resuspended in complete culture medium and counted for cell number and cell viability. Upon confirmation of cell viability greater than 80%, seeding of PBMC into 48-well culture plate (Catalog#150687; Thermo Fisher Scientific Inc.) in the cell density of 1 × 10⁶ cell/per well containing 0.5 mL of complete culture medium was performed. Culture plate was incubated at 37°C, 5% CO₂ overnight. On Day 2, equal volume of antigen and IL-2, IL-7 containing medium were added into each well. Incubate the plates at 37°C until use. On Day 5, refresh of the medium with antigen and cytokines was performed, then incubate the plates at 37°C until use. On Day 8, seeding the cells and PBMC was collected and transferred into new 48-well plate at 37°C, 5% CO₂.

### Measurement of total IgM

Total IgM induction status was evaluated by commercially available total IgM ELISPOT kit (Cellular Technology Ltd.). On Day 10, a 96-well plate coated with human Ig capture antibody was incubated at 4°C overnight. Plate was washed once with PBS and used for seeding of the treated PBMC at 2.5 × 10⁴ cells per well density. Incubate the plates at 37°C overnight, avoid shaking. On Day 12, plate was washed twice with PBS, followed by two wash cycles with 0.05% PBST (0.05% Tween-20 in PBS). 80 µL of biotin-labeled anti-IgM detection antibody were added into each well and incubated at room temperature for 2 hours. Plate was washed three times with 0.05% PBST and 80 µL of Tertiary solution were added into each well. Incubation at room temperature was conducted for 1 hour. Plate was washed twice with 0.05% PBST followed by two wash cycles with ddH₂O. Plate was added with 80 µL of mixed blue developer solution per well, and incubated at room temperature for 15 minutes while avoiding light exposure. Plate was rinsed with tap water and put in inverted position to air-dry. Imaging of the 96-well plate was performed using a CCD video camera, and the spot number was determined by ImmunoSpot^{®} software (Cellular Technology Ltd.).

### Measurement of anti-Globo H IgM

Anti-Globo H IgM was evaluated by commercially available total IgM ELISPOT kit (Cellular Technology Ltd.). On Day 10, a flat bottom 96-well plate (Catalog #3917; Coming Incorporated) was coated with 0.2 µg Globo H-lipid (C₇₈H₁₄₄N₂O₃₃, MW 1637.9716) in 100 µL ethanol per well. Plate was incubated at room temperature overnight where the ethanol was completely evaporated. Plate was added with 200 µL of complete culture medium per well and incubated at room temperature for 1 hour.

Plate was used for seeding of the treated PBMC at 2 × 10⁵ cells per well density. Incubate the plates at 37°C overnight, avoid shaking. On Day 12, plate was washed twice with PBS, followed by two wash cycles with 0.05% PBST. Each well of the plate was added with 100 µL of filtered goat anti-human IgM-biotin antibody (Catalog#109-067-043; Jackson ImmunoResearch Inc.) solution in 1% BSA dissolved in PBS at 1:1000 dilution. Plate was incubated at room temperature for 2 hours. Plate was washed with 0.05% PBST three times. Each well was added with 100 µL of streptavidin-HRP (Catalog#DY998; R&D Systems, Inc.) in 1% BSA buffer at 1:200 dilution. Plate was incubated at room temperature for 1 hour. Two wash steps with 0.05% PBST followed by two washes of ddH₂O were performed. Plate was added with 100 µL of mixed substrate solution (Catalog#CTL-STR10; Cellular Technology Ltd.) per well, and incubated at room temperature for 15 minutes while avoiding light exposure. Plate was rinsed with tap water and put in inverted position to air-dry. Imaging of the 96-well plate was performed using a CCD video camera, and the spot number was determined by ImmunoSpot^{®} software (Cellular Technology Ltd.).

### Measurement of IFN-γ

On Day 11, PBMC cultured supernatant were collected into a 15 mL tube (Catalog #430791; Corning Incorporated), and centrifuged at 400g at room temperature for 5 minutes. Supernatant was transferred into a new 1.5 mL microtube (Cataloge #MCT-150-C; Coming Incorporated), and stored at -80°C. IFN-γ was measured by MSD V-PLEX human proinflammatory cytokine kit (Catalog #K151A0H-2; Meso Scale Diagnostic, LLC). The experimental procedures were performed according to manufacturer's instructions. The results were acquired by MESO SECTOR S600 (Meso Scale Diagnostic, LLC).

### Example 2: The modified anti-Globo H IgM ELISPOT assay

The conventional and modified ELISPOT assay were compared in parallel (Group A: No antigen; Group B: B-Poly-S^{™} (Catalog #CTL-hBPOLYS-200; Cellular Technology Ltd.); Group C: OBI-821 adjuvant; Group D: OBI-821 adjuvant+OBI-822 vaccine; Group E: medium only). Conventional and modified ELISPOT assays were performed using PVDF bottom 96-well plate (Catalog #MSIPS4510; Merck KGaA) and flat white bottom 96-well plate, respectively. The condition of coating and seeding of PMBC cell number for both assays were identical. **Figure 2** illustrates higher visibility and sensitivity of modified ELISPOT assay when compared to conventional method.

### Example 3: The accuracy of modified anti-Globo H IgM ELISPOT

The ExpiCHO-S cells (Catalog #A29127; Thermo Fisher Scientific Inc.) were cultured in ExpiCHO expression medium. When cells density reached 4 × 10⁶ to 6 × 10⁶ viable cells/mL, 1.8 × 10⁸ cells were co-transfected by ExpiFectamine CHO transfect kit with 15 µg of plasmid Zsgreen and anti-human GloboH-IgM antibody in 30 mL ExpiCHO expression medium. After 8 days of transfection, cells were collected for assay. Three types of cells, including CHO cell only, CHO cells transfected with Zsgreen fluorescent protein sequence (SEQ ID No. 1), and CHO cells co-transfected with Zsgreen and human Globo H IgM, were seeded for anti-Globo H IgM ELISPOT assay,. **Figure 3A** illustrates the transfection efficiency of 55 to 61% on the seeding day (Day 11) by Flow Cytometry. **Figure 3B** and **3C** illustrate that only the CHO cells co-transfected with Zsgreen and human Globo H IgM had detectible spots, which confirm the selectivity and accuracy of the modified Globo H IgM ELISPOT assay.

### Example 4: Serum anti-Globo H IgM or IgG concentration of OBI-833/821 vaccinated patients

Patients were immunized with OBI-833/821 in a phase 1 clinical study (Trial Identifier #NCT02310464). Anti-Globo H IgM and IgG concentration were measured by glycan chip. Maximum serum Globo H IgM and IgG concentration (µg/mL) of each sample are summarized in **Table 1.** Patients' anti-Globo H IgM and IgG concentration in serum will be used to compare with *ex vivo* immunogenicity assay results.

**Table 1. Serum anti-Globo H IgM or IgG concentration of OBI-833/821 vaccinated patients.**

| | **OBI-833/821** | |
|---|---|---|
| **Patient ID** | **Clinical Globo H IgM (µg/mL)** | **Clinical Globo H IgG (µg/mL)** |
| 001-004 | 6.19 | 9.11 |
| 034-002 | 57.21 | 5.06 |
| 034-004 | 242.81 | 4.15 |
| 034-006 | 10.63 | 13.72 |
| 034-007 | 25.36 | <LLOQ* |

| | | |
|---|---|---|
| *LLOQ: Lower Limit of Quantification. | | |

### Example 5: Ex vivo immunogenicity assay result from patient PBMC

### Total IgM and Globo H IgM response

Patient PBMC was collected and cryopreserved prior to OBI-833/821 vaccination. *Ex vivo* immunogenicity assay was performed following protocols illustrated in Figure 1. PBMC from known healthy donor was used as experimental control. Spot number and fold change value normalized with no-antigen treatment group of total IgM and anti-Globo H IgM by ELISPOT assay are summarized in **Table 2.**

**Table 2. Total IgM and anti-Globo H IgM ELISPOT result from patient PBMC.**

| | **Spot Number** | | | | **Fold changes** | | | |
|---|---|---|---|---|---|---|---|---|
| | **Total IgM ELISpot** | | **Globo H IgM ELISpot** | | **Total IgM ELISpot** | | **Globo H IgM ELISpot** | |
| **Patient ID** | **No Ag** | **OBI-833+ 821** | **No Ag** | **OBI-833+ 821** | **No Ag** | **OBI-833+ 821** | **No Ag** | **OBI-833+ 821** |
| 001-004 | 21 | 31.5 | 8 | 78 | 1 | 1.50 | 1 | 9.75 |
| 034-002 | 5.3 | 42 | 38 | 65 | 1 | 7.92 | 1 | 1.71 |
| 034-004 | 17 | 179 | 9.6 | 123 | 1 | 10.53 | 1 | 12.81 |
| 034-006 | 53 | 111.6 | 14.3 | 30 | 1 | 2.11 | 1 | 2.10 |
| 034-007 | 22.5 | 46.6 | 10.5 | 18.5 | 1 | 2.07 | 1 | 1.76 |

Total IgM and Globo H IgM responses from five patients' PBMC are illustrated in **Figure 4A** and **4B****,** respectively. The results indicated that with *ex vivo* immunogenicity culturing procedures, various levels of total IgM and/or Globo H IgM were induced using patients' PBMC.

### IFN-γ response

In addition to antibody responses, cytokine production, such as IFN-γ, was evaluated using *ex vivo* immunogenicity culturing procedures. IFN-γ concentration (pg/mL) detected in the culture supernatant and fold change normalized with no-antigen treatment group are illustrate in **Table 3** and **Figure 4C****,** respectively. The results indicated that with *ex vivo* immunogenicity culturing procedures, various levels of IFN-γ were induced using patients' PBMC

**Table 3. IFN-γ induction result from patient PBMC.**

| | **IFN-γ (pg/mL)** | | **Fold changes** | |
|---|---|---|---|---|
| **Patient ID** | **No Ag** | **OBI-833+821** | **No Ag** | **OBI-833+821** |
| 001-004 | 18795 | 23142 | 1 | 1.23 |
| 034-002 | 10054 | 24715 | 1 | 2.46 |
| 034-004 | 22915 | 99184 | 1 | 4.33 |
| 034-006 | 15754 | 35523 | 1 | 2.25 |
| 034-007 | 4396 | 8680 | 1 | 1.97 |

### Example 6: Correlation between ex vivo immunogenicity assay results and clinical response

The correlation of patients' maximum serum anti-Globo H IgM concentration (µg/mL) in comparison with either total IgM, anti-Globo H IgM, and IFN-γ cytokine induction are presented in **Figure 5A****,** **5B****, and** **5C****,** respectively. ELISPOT result as well as IFN-γ induction are shown in fold change normalized with no-antigen treatment group. Positive correlation between results and patients' serum anti-Globo H IgM level were observed.

In addition, correlation of patients' maximum serum anti-Globo H IgG concentration (µg/mL) in comparison with either total IgM, anti-Globo H IgM, and IFN-γ cytokine induction are presented in **Figure 6A****,** **6B****, and** **6C** respectively. Overall, results showed more obvious correlation with anti-Globo H IgM than anti-Globo H IgG. Patients may have delayed IgG response where correlation cannot be observed yet.

Unless defined otherwise, all technical and scientific terms and any acronyms used herein have the same meanings as commonly understood by one of ordinary skill in the art in the field of this invention. Although any compositions, methods, kits, and means for communicating information similar or equivalent to those described herein can be used to practice this invention, the preferred compositions, methods, kits, and means for communicating information are described herein. All references cited herein and the discussion of those references is intended merely to summarize the assertions made by their authors. No admission is made that any reference (or a portion of any reference) is relevant prior art. Applicants reserve the right to challenge the accuracy and pertinence of any cited reference.

## Claims

1. A method for evaluating responsivity of a subject to a carbohydrate antigen or its immunogenic fragment, comprising:
(a) obtaining a peripheral blood mononuclear cell from the subject;
(b) exposing the cell *ex-vivo* with the carbohydrate antigen or its immunogenic fragment in an Enzyme-Linked ImmunoSpot assay performed by using carbohydrate antigen-coated plate; and
(c) determining a biological activity according to the assay to evaluate the responsivity,
wherein the carbohydrate antigen or its immunogenic fragment is a Globo series antigen, and wherein the carbohydrate antigen-coated plate is a plate without PVDF.

2. The method of claim 1, wherein the Globo series antigen is stage-specific embryonic antigen-4 (SSEA-4), stage-specific embryonic antigen-3 (SSEA-3) or Globo H.

3. The method of claim 1, wherein the carbohydrate antigen or its immunogenic fragment is conjugated with a carrier protein, wherein, preferably, the carrier protein comprises KLH (Keyhole limpet hemocyanin) or DT-CRM 197 (diphtheria toxin cross-reacting material 197).

4. The method of claim 1, wherein the carbohydrate antigen is formulated with an adjuvant into a cancer vaccine, wherein, preferably, the adjuvant comprises QS-21, saponin, Freund's adjuvant, α-galactosyl-ceramide (α-GalCer) adjuvant, OBI-821, OBI-834, or a combination thereof.

5. The method of claim 4, wherein the cancer is a Globo series antigen expressing cancer, wherein, preferably, the Globo series antigen expressing cancer is sarcoma, skin cancer, leukemia, lymphoma, brain cancer, glioblastoma, lung cancer, breast cancer, oral cancer, head-and-neck cancer, nasopharyngeal cancer, esophagus cancer, stomach cancer, liver cancer, bile duct cancer, gallbladder cancer, bladder cancer, pancreatic cancer, intestinal cancer, colorectal cancer, kidney cancer, cervix cancer, endometrial cancer, ovarian cancer, testicular cancer, buccal cancer, oropharyngeal cancer, laryngeal cancer or prostate cancer.

6. The method of claim 4, wherein the cancer vaccine is OBI-822/821, OBI-833/821, OBI-833/834, OBI-866/821 or OBI-866/834.

7. The method of claim 1, wherein the biological activity comprises production of IgM, IgM against the carbohydrate antigen, cytokine, or a combination thereof, wherein, preferably, the cytokine is human growth hormone, parathyroid hormone, glycoprotein hormone, fibroblast growth factor, TNF-α, TNF-β, vascular endothelial growth factor, integrin, thrombopoietin (TPO), nerve growth factor, platelet-growth factor, TGF-α, TGF-β, interferon-α, interferon-β, interferon-γ, macrophage-CSF (M-CSF), granulocyte-macrophage-CSF (GM-CSF), granulocyte-CSF (G-CSF), IL-1, IL-1α, IL-2, IL-3, IL-4, IL-5, IL-6, IL-7, IL-8, IL-9, IL-10, IL-11 or IL-12.

8. The method of claim 1, wherein the biological activity is determined by spot number of the assay.

9. The method of claim 1, wherein the responsivity is evaluated by comparing the biological activity with a threshold value and the subject is identified as responsive to the carbohydrate antigen if the biological activity exceeds the threshold value.

## Patentansprüche

1. Verfahren zur Bewertung der Ansprechbarkeit eines Patienten auf ein Kohlenhydrat-Antigen oder seines immunogenen Fragments, umfassend:
(a) Erhalten einer mononuklearen Zelle aus Peripherblut von dem Patienten;
(b) Aussetzen der Zelle *ex-vivo* gegenüber dem Kohlenhydrat-Antigen oder seinem immunogenen Fragment in einem Enzym-verknüpften ImmunoSpot-Assay, der unter Verwendung einer mit dem Kohlenhydrat-Antigen beschichteten Platte durchgeführt wird; und
(c) Bestimmen einer biologischen Aktivität gemäß dem Assay zum Bewerten der Ansprechbarkeit,
wobei das Kohlenhydrat-Antigen oder sein immunogenes Fragment ein Antigen der Globo-Reihe ist, und wobei die mit dem Kohlenhydrat-Antigen beschichtete Platte eine Platte ohne PVDF ist.

2. Verfahren nach Anspruch 1, wobei das Antigen der Globo-Reihe ein stadium-spezifisches embryonisches Antigen-4 (SSEA-4), ein stadium-spezifisches embryonisches Antigen-3 (SSEA-3) oder Globo H ist.

3. Verfahren nach Anspruch 1, wobei das Kohlenhydrat-Antigen oder sein immunogenes Fragment mit einem Trägerprotein konjugiert ist, wobei, bevorzugt, das Trägerprotein KLH (Keyhole Limpet Hemocyanin) oder DT-CRM 197 (Diphtherietoxin-kreuzreagierendes Material 197) umfasst.

4. Verfahren nach Anspruch 1, wobei das Kohlenhydrat-Antigen mit einem Adjuvans zu einem Krebsvakzin formuliert wird, wobei, bevorzugt, das Adjuvans QS-21, Saponin, Freundsches Adjuvans, α-Galactosyl-Ceramid (α-GalCer) Adjuvans, OBI-821, OBI-834 oder eine Kombination davon umfasst.

5. Verfahren nach Anspruch 4, wobei der Krebs ein Globo-Reihe-Antigen-exprimierender Krebs ist, wobei, bevorzugt, der Globo-Reihe-Antigen-exprimierende Krebs Sarkom, Hautkrebs, Leukämie, Lymphom, Gehirnkrebs, Glioblastom, Lungenkrebs, Brustkrebs, Mundkrebs, Kopf- und Halskrebs, Nasen-Rachen-Krebs, Speiseröhrenkrebs, Magenkrebs, Leberkrebs, Gallengangkrebs, Gallenblasenkrebs, Blasenkrebs, Pankreaskrebs, Darmkrebs, Kolorektal-Krebs, Nierenkrebs, Gebärmutterhalskrebs, Endometrialkrebs, Eierstockkrebs, Hodenkrebs, Mundhöhlenkrebs, Oropharynxkrebs, Kehlkopfkrebs oder Prostatakrebs ist.

6. Verfahren nach Anspruch 4, wobei das Krebsvakzin OBI-822/821, OBI-833/821, OBI-833/834, OBI-866/821 oder OBI-866/834 ist.

7. Verfahren nach Anspruch 1, wobei die biologische Aktivität die Produktion von IgM, IgM gegen das Kohlenhydrat-Antigen, Zytokin oder eine Kombination davon umfasst, wobei, bevorzugt, das Zytokin humanes Wachstumshormon, Parathormon, Glykoproteinhormon, Fibroblastenwachstumsfaktor, TNF-α, TNF-β, vaskulärer endothelialer Wachstumsfaktor, Integrin, Thrombopoietin (TPO), Nervenwachstumsfaktor, Blutplättchen-Wachstumsfaktor, TGF-α, TGF-β, Interferon-α, Interferon-β, Interferon-γ, Makrophagen-CSF (M-CSF), Granulozyten-Makrophagen-CSF (GM-CSF), Granulozyten-CSF (G-CSF), IL-1, IL-1α, IL-2, IL-3, IL-4, IL-5, IL-6, IL-7, IL-8, IL-9, IL-10, IL-11 oder IL-12 ist.

8. Verfahren nach Anspruch 1, wobei die biologische Aktivität anhand der Anzahl der Spots des Assay bestimmt wird.

9. Verfahren nach Anspruch 1, wobei die Ansprechbarkeit durch Vergleich der biologischen Aktivität mit einem Schwellenwert bewertet wird und der Patient als auf das Kohlenhydrat-Antigen ansprechend identifiziert wird, wenn die biologische Aktivität den Schwellenwert überschreitet.

## Revendications

1. Procédé pour évaluer la sensibilité d'un sujet à un antigène carbohydrate ou son fragment immunogène, comprenant :
(a) l'obtention d'une cellule mononucléaire du sang périphérique à partir du sujet ;
(b) l'exposition de la cellule ex *vivo* à l'antigène carbohydrate ou son fragment immunogène dans un dosage ELISpot (Enzyme-Linked ImmunoSpot) réalisé en utilisant une plaque revêtue d'antigène carbohydrate ; et
(c) la détermination d'une activité biologique en fonction du dosage afin d'évaluer la sensibilité,
dans lequel l'antigène carbohydrate ou son fragment immunogène est un antigène de la série Globo, et dans lequel la plaque revêtue d'antigène carbohydrate est une plaque sans PVDF.

2. Procédé selon la revendication 1, dans lequel l'antigène de la série Globo est l'antigène embryonnaire spécifique au stade 4 (SSEA-4), l'antigène embryonnaire spécifique au stade 3 (SSEA-3) ou Globo H.

3. Procédé selon la revendication 1, dans lequel l'antigène carbohydrate ou son fragment immunogène est conjugué à une protéine porteuse, dans lequel, de préférence, la protéine porteuse comprend la KLH (hémocyanine de patelle) ou le DT-CRM 197 (matériel 197 à réaction croisée avec la toxine diphtérique).

4. Procédé selon la revendication 1, dans lequel l'antigène carbohydrate est formulé avec un adjuvant dans un vaccin anticancéreux, dans lequel, de préférence, l'adjuvant comprend le QS-21, une saponine, un adjuvant de Freund, un adjuvant α-galactosyl-céramide (α-GalCer), l'OBI-821, l'OBI-834, ou une combinaison de ceux-ci.

5. Procédé selon la revendication 4, dans lequel le cancer est cancer exprimant un antigène de la série Globo, dans lequel, de préférence, le cancer exprimant un antigène de la série Globo est un sarcome, le cancer de la peau, une leucémie, un lymphome, le cancer du cerveau, un glioblastome, le cancer du poumon, le cancer du sein, le cancer de la cavité orale, le cancer de la tête et du cou, le cancer du nasopharynx, le cancer de l'œsophage, le cancer de l'estomac, le cancer du foie, le cancer des voies biliaires, le cancer de la vésicule biliaire, le cancer de la vessie, le cancer du pancréas, le cancer de l'intestin, le cancer colorectal, le cancer du rein, le cancer du col de l'utérus, le cancer de l'endomètre, le cancer de l'ovaire, le cancer du testicule, le cancer de la bouche, le cancer de l'oropharynx, le cancer du larynx ou le cancer de la prostate.

6. Procédé selon la revendication 4, dans lequel le vaccin anticancéreux est l'OBI-822/821, l'OBI-833/821, l'OBI-833/834, l'OBI-866/821 ou l'OBI-866/834.

7. Procédé selon la revendication 1, dans lequel l'activité biologique comprend la production d'une IgM, d'une IgM dirigée contre l'antigène carbohydrate, d'une cytokine, ou d'une combinaison de celles-ci, dans lequel, de préférence, la cytokine est l'hormone de croissance humaine, l'hormone parathyroïdienne, l'hormone glycoprotéique, le facteur de croissance des fibroblastes, le TNF-α, le TNF-β, le facteur de croissance de l'endothélium vasculaire, une intégrine, la thrombopoïétine (TPO), le facteur de croissance des nerfs, le facteur de croissance des plaquettes, le TGF-α, le TGF-β, l'interféron-α, l'interféron-β, l'interféron-γ, le facteur de stimulation des colonies de macrophages (M-CSF), le facteur de stimulation des colonies de granulocytes-macrophages (GM-CSF), le facteur de stimulation des colonies de granulocytes (G-CSF), l'IL-1, l'IL-1α, l'IL-2, l'IL-3, l'IL-4, l'IL-5, l'IL-6, l'IL-7, l'IL-8, l'IL-9, l'IL-10, l'IL-11 ou l'IL-12.

8. Procédé selon la revendication 1, dans lequel l'activité biologique est déterminée par le nombre de spots de l'essai.

9. Procédé selon la revendication 1, dans lequel la sensibilité est évaluée en comparant l'activité biologique avec une valeur seuil et le sujet est identifié comme sensible à l'antigène carbohydrate si l'activité biologique excède la valeur seuil.
